# EUROPEAN PATENT APPLICATION

(11) **EP 4 487 698 A1**
(43) Date of publication of application: **08.01.2025**
(21) Application number: 24186633.4
(22) Date of filing: 04.07.2024
(51) Int. Cl.: A23L 33/105, A23L 33/15, A23L 33/175, A61K 31/185, A61K 31/19, A61K 31/194, A61K 31/197, A61K 31/198, A61K 31/401, A61K 31/4172, A61K 31/455

(54) **A MICRONUTRIENT COMPOSITION TO PREVENT AND REVERSE PROTEIN GLYCATION DURING OXIDATIVE STRESS IN HUMAN**

(30) Priority: 05.07.2023 US 202318218178
(71) Applicant: Rath, Matthias W., Henderson, NV 89044 (US)
(72) Inventor: Rath, Matthias W., Henderson, NV Nevada 89044 (US); Niedzwiecki, Aleksandra, Henderson, 89044 (US); Chatterjee, Madhurima, Santa Clara, 95054 (US)
(74) Representative: Altmann Stößel Dick Patentanwälte PartG mbB

(57) **Abstract**

The micronutrient composition is a combination of at least 6 ingredients, for example alpha-Ketoglutaric Acid, Niacinamide and/or Niacin, L-Proline, L-Glutamine, L-Citrulline and Sodium Pyruvate, preferably at least 11 ingredients, for example alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate, Magnesium Citrate and Methionine, more preferably all of the ingredients which are selected from the group consisting of: alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate and/or Magnesium Citrate, Methionine and/or S- Adenosyl-Methionine (SAMe), Methylsulfonylmethane (MSM), and Wormwood powder and/or extract. The composition is effectively used to treat diseases due to oxidative stress. More specifically, the micronutrient composition is effective in reversing glycation in diabetes and thus in treating diabetes.

## Description

### FIELD OF INVENTION

The instant invention is focused on a micronutrient composition that prevents and reverses protein glycation created due to oxidative stress in human.

### BACKGROUND

Advanced Glycation End Products (AGEs) are modifications of proteins or lipids which form in vivo in hyperglycemic environments and during aging and contribute to the pathophysiology of diabetes, cardiovascular disease, Alzheimer's, inflammation to overall accelerated aging of tissues. Many of AGEs negative effects occur via AGE activation of a cell receptor called Receptor of Advanced Glycation End products (RAGE).

AGEs modified proteins or lipids are a result of nonenzymatic glycation and oxidation after their contact with sugars, notably fructose and glucose. The AGE formation process, or the Maillard reaction, begins from Schiff bases and the Amadori product, produced by the reaction of the carbonyl group of a reducing sugar, like glucose, with proteins, lipids, and nucleic acid amino groups. These highly reactive intermediate carbonyl groups, known as α-dicarbonyls or oxoaldehydes have the ability to react with amino, sulfhydryl, and guanidine functional groups in proteins resulting in cross-linking and denaturation of proteins. In addition, the α-dicarbonyls can react with lysine and arginine functional groups on proteins, leading to the formation of stable AGE compounds, such as *N*^{ε}-(carboxymethyl)lysine (CML), which are nonfluorescent AGEs. CMLs also form in vitro from LDL incubated with copper ions and glucose and therefore are believed to be both lipid and protein adducts. Once AGEs are formed, they are considered nearly irreversible. Therefore, there is an urgent objective to develop effective, economic and side effects free approaches to protect cells against oxidative stress.

### SUMMARY

The objective is achieved by a micronutrient composition, comprising or consisting of at least 6 ingredients, for example alpha-Ketoglutaric Acid, Niacinamide and/or Niacin, L-Proline, L-Glutamine, L-Citrulline and Sodium Pyruvate, preferably at least 11 ingredients, for example alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate, Magnesium Citrate and Methionine, more preferably all of the ingredients which are selected from the group consisting of:
alpha-Ketoglutaric Acid, Niacinamide and/or Niacin, L-Proline, L-Glutamine, L-Citrulline,
Sodium Pyruvate, Pantothenic Acid, L-Cysteine and/or N-acetyl Cysteine, Magnesium Malate and/or Magnesium Citrate, Methionine and/or S- Adenosyl-Methionine (SAMe),
Methylsulfonylmethane (MSM), and Wormwood powder and/or extract,
the micronutrient composition optionally additionally containing one or a combination of pharmaceutically acceptable carrier or excipient or liquefied propellant or buffer or pH regulator or stabilizer or coating or flavoring agent or formulation adjuvant,
the micronutrient composition optionally being formulated as a tablet, coated tablet, capsule, pill, intranasal, lozenge, emulsion, pastille, suppository, paste or injectable solution.

Thus, the micronutrient composition of the present invention comprising or consisting of at least 6, preferably at least 11, more preferably at least 14 ingredients which are selected from the group consisting of:
alpha-Ketoglutaric Acid, Niacinamide and/or Niacin, L-Proline, L-Glutamine, L-Citrulline,
Sodium Pyruvate, Pantothenic Acid, L-Cysteine and/or N-acetyl Cysteine, Magnesium Malate and/or Magnesium Citrate, Methionine and/or S- Adenosyl-Methionine (SAMe),
Methylsulfonylmethane (MSM), and Wormwood powder and/or extract,
and which preferably are selected from the group consisting of:
   alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, Methionine, S- Adenosyl-Methionine (SAMe), Methylsulfonylmethane (MSM), and Wormwood powder or extract.

The micronutrient composition of the present invention can comprise or consist of 6-20, preferably 11-20, more preferably 1-20 ingredients which are selected from the above group

The micronutrient composition of the present invention can, as a preferred alternative, comprise or consist of 6-14, preferably 11-14, more preferably 14 ingredients which are selected from the above group.

The micronutrient composition, comprising or consisting of at least 6 ingredients, preferably alpha-Ketoglutaric Acid, Niacinamide and/or Niacin, L-Proline, L-Glutamine, L-Citrulline and Sodium Pyruvate, more preferably comprising or consisting of alpha-Ketoglutaric Acid, Niacinamide or Niacin, L-Proline, L-Glutamine, L-Citrulline and Sodium Pyruvate, thus containing 6 micronutrients, is denoted as Core in the following.

The micronutrient composition, comprising or consisting of at least 11 ingredients, for example alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate, Magnesium Citrate and Methionine, more preferably comprising or consisting of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate and Methionine, thus containing 11 micronutrients, is denoted as Mix in the following.

The micronutrient composition, comprising or consisting of at least 14 ingredients, preferably alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate, Magnesium Citrate, Methionine, S- Adenosyl-Methionine (SAMe), Methylsulfonylmethane (MSM), and Wormwood powder and/or extract, more preferably comprising or consisting of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, Methionine, S- Adenosyl-Methionine (SAMe), Methylsulfonylmethane (MSM), and Wormwood powder or extract, thus containing 14 micronutrients, is denoted the complete micronutrient composition in the following.

Table 1 lists the ingredients combined in the Core (bold font) and Mix (Bold and regular font) that are preferred compositions according to the present invention.

Preferred is a micronutrient composition, in which the ingredients, if present, are contained in the following amounts: alpha-Ketoglutaric Acid 10 mg-30000 mg, Niacinamide and/or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder and/or extract 1 mg-10000 mg.

Preferably, the micronutrient composition, consists of alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate and/or Magnesium Citrate, and Methionine, and optionally additionally one or a combination of pharmaceutically acceptable carrier or excipient or liquefied propellant or buffer or pH regulator or stabilizer or coating or flavoring agent.

Preferred is a micronutrient composition, in which the ingredients, if present, are contained in the following amounts: alpha-Ketoglutaric Acid 10 mg-30000 mg, Niacinamide and/or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, and Methionine 10 mg-20000 mg.

Preferred is a micronutrient composition , wherein the dosage form is one oral capsule once a day, twice a day or three times a day.

Preferred is a micronutrient composition , wherein the formulation adjuvant is selected from the group consisting of a binder, a disintegrant, a lubricant, a coloring agent, a sweetening agent, a flavoring agent, and mixtures thereof.

The objective is achieved by the use of the above micronutrient composition in a pharmaceutical composition.

The objective is achieved by a pharmaceutical composition, containing the above micronutrient composition and optionally a pharmaceutically acceptable carrier.

The objective is achieved by the above pharmaceutical composition for use in the prevention or treatment of diabetes, cardiovascular disease, Alzheimer's disease or inflammation, preferably diabetes.

The objective is achieved by a process for preparing the above micronutrient composition or the above pharmaceutical composition by mixing the ingredients of the micronutrient composition or the pharmaceutical composition and optionally bringing the mixture into the desired dosage form.

Preferably, the process comprises mixing the ingredients, if present, in the following amounts: alpha-Ketoglutaric Acid 10 mg-30000 mg, Niacinamide and/or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder and/or extract 1 mg-10000 mg.

Preferably, the process comprises formulating the micronutrient composition as a tablet, coated tablet, capsule, pill, intranasal, lozenge, emulsion, pastille, suppository, paste or injectable solution.

In the instant disclosure various combination of micronutrients as a composition were used to treat and prevent, AGEs formation in patients suffering from not only diabetes but other diseases wherein protein turnover is inhibited. In one embodiment a physiological dose for a mammal was calculated based on daily consumption. The formula was packaged in drug formulation for easy consumption.

In one embodiment, the micronutrient composition comprises of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, Methionine, S-adenosyl-methionine (SAMe), Methylsulfonylmethane (MSM), and Wormwood powder or extract. In one embodiment the micronutrient composition comprises of alpha-Ketoglutaric Acid 10 mg-30000 mg, Niacinamide or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder or extract 1 mg-10000 mg.

In one embodiment, a micronutrient composition shown as Mix in figures comprises of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, and Methionine. In one embodiment the micronutrient composition comprises of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg.

In one embodiment, a method of treating a human suffering from a disease that manifests due to oxidative stress by administering micronutrient composition is disclosed. In another embodiment, micronutrient composition (Mix) comprising of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg.

In one embodiment, a method of treating a human suffering from a disease that manifests due to oxidative stress by administering micronutrient composition is disclosed. In another embodiment, micronutrient composition (Core mix) consisting of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, and Sodium Pyruvate 10 mg-50000 mg.

In one embodiment, micronutrient composition, the Mix and the core mix are used as a pharmaceutical composition and administered to a human suffering from oxidative stress induced specific diseases such as diabetes, cardiovascular disease, Alzheimer's and inflammation to overall accelerated aging of tissues. The micronutrient composition, the Mix and the core mix are administered in various forms to a human to treat specific diseases, such as diabetes, cardiovascular disease, Alzheimer's and inflammation to overall accelerated aging of tissues.

### BRIEF DESCRIPTION OF DRAWINGS

Example embodiments are illustrated by way of example and not limitation in the figures of the accompanying drawings, in which like references indicate similar elements and in which:
Figure 1 shows micronutrient composition as Mix and Core having simultaneous pleiotropic beneficial effects in metabolic aspects of diabetes.
Figure 2 shows is a prior art showing the chemical reaction for glycation reversal assay.
Figure 3 shows reversal of carboxymethyl-lysine (CML) to native lysine in the presence of individual compounds and their combinations (Core and the Mix).
Figure 4 shows effects of individual natural compounds and their combinations as Mix and Core on AGE-BSA formation in the presence of glucose.
Figure 5 shows effects of individual natural compounds and their combinations as Mix and Core on AGE-BSA formation in the presence of fructose.
Figure 6 shows effects of individual ingredients, and their combinations (Core and Mix) on Succinate Dehydrogenase (SDHA) levels in the heart muscle cells.
Figure 7 shows effects of individual ingredients, and their combinations (Core and the Mix) on Cox-1 levels in the heart muscle cells.
Figure 8 shows protection of microglia cells against oxidative stress generated by hydrogen peroxide by individual compounds and their combinations (Core and the Mix).

Others features of the present embodiments will be apparent from the accompanying drawings and from the detailed description that follows.

### DEATILED DESCRIPTION

The instant disclosure shows various combinations of individual components of micronutrient composition, preferably Mix and Core and their effect on preventing and treating oxidative stress related functions in specific diseases such as diabetes, cardiovascular disease, Alzheimer's and inflammation to overall accelerated aging of tissues. The micronutrient composition as a Mix comprises or consists of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, and Methionine. The physiological range of micronutrient composition for Mix is alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg.

The micronutrient composition as a Core comprises of alpha-Ketoglutaric Acid, Niacinamide or Niacin, L-Proline, L-Glutamine, L-Citrulline, Sodium Pyruvate, and their physiological dose comprise in the range of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, and Sodium Pyruvate 10 mg-50000 mg.

We tested several natural compounds individually and as mixtures to address their efficacy in both prevention and reversal of AGE formation in vitro. The AGE reversal assay was based on a method to convert N^{ε}-(carboxymethyl)lysine (CML) which is an AGE product and a known physiological ligand of RAGE to L-Lysine. Thus, the conversion of CML to L-Lysine would represent the reversal of the glycation modification. The assay was adapted from a publication by Kim et al where they used a modified E. coli enzyme to cleave CML. We applied natural compounds, amino acids and Krebs cycle components, to obtain the same effects.

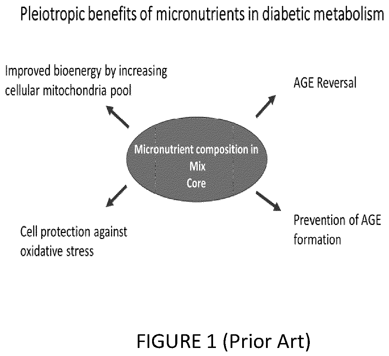

In addition, using an established protocol for AGE formation we showed that combinations of natural compounds (Mix and Core) and, to a lesser degree, individual test ingredients were effective in preventing glycation of Bovine Serum Albumin (BSA) by both glucose and fructose over a 5 day incubation period. We also showed beneficial effects of the micronutrients at the cellular level in important aspects associated with hyperglycemia and diabetes, which include oxidative stress, nerve damage and mitochondrial dysfunction. Our results show that individual ingredients work synergistically to promote mitobiogenesis in the muscle cells and protect microglial cells from death caused by oxidative stress. Mitobiogenesis assay specifically detects the Cox-1 activity (in respiratory chain Complex IV) and SDH (Succinate Dehydrogenase) subunit A which is another mitochondrial membrane protein as well as a tumor suppressor. Figure 1 shows a general view of micronutrient composition has pleiotropic beneficial effects in metabolic aspects of diabetes.

Materials: N-carboxymethyllysine (CML) was purchased from Cayman Chemical Company (Michigan, USA). Sources of other test compounds are listed in Table 1 and Table 2:

**Table 1. Test ingredients combined in the Core (bold font) and Mix (Bold and regular font)**

| **Name** | **Company** | **Batch No** |
|---|---|---|
| **alpha-Ketoglutaric Acid** | **Double Wood Supplements. Pennsylvania, USA** | **2135700** |

| **Niacinamide** | **Nutricost. Utah, USA** | **21121601** |
|---|---|---|
| **L-Proline** | **MilliporeSigma. Massachusetts, USA** | **119h0342** |
| **L-Glutamine** | **Bulk Supplements. Nevada,USA** | **2100811** |
| **L-Citrulline** | **Bulk Supplements. Nevada, USA** | **2113910** |
| **Sodium Pyruvate** | **Research Products International. Illinios, USA** | **12538719** |
| | | |
| Pantothenic Acid | Vitamatic. Altea, Spain | P2160051 |
| L-Cysteine | MilliporeSigma. Massachusetts, USA | SLBQ0450V |
| Magnesium Malate | NOW. Illinois, USA | 3243235 |
| Magnesium Citrate | NOW. Illinois, USA | 3237352 |
| Methionine | MilliporeSigma. Massachusetts, USA | SLBF0519V |

**Table 2. Additional ingredients evaluated here**

| | | |
|---|---|---|
| S- adenosyl-methionine | Cayman Chemical Company | 0461501-21 |
| Methylsulfonylmethane (MSM) Powder | Powder City PA, USA | 7515 |
| Wormwood herb. Powder (*Artemisia adsinthium*) | Monterey Bay Spice Company. CA, USA | 16L347-137 |

All ingredients were solubilized in Dimethylsulfoxide (DMSO) from MilliporeSigma (Massachusetts, USA). The complete micronutrient composition comprises of 14 ingredients. The Mix was formed by combining all 11 ingredients listed in Table 1 in equal amounts. A subset for the Mix ingredients designated as Core is formed by 6 ingredients. Other compounds tested in the study but not included in the Mix and Core are listed in Table 2.

Other reagents used were 2,4-dinitrophenylhydrazine (2,4-DNPH) from MilliporeSigma (Massachusetts, USA), Hydrochloric Acid from Thermo Fisher Scientific (Massachusetts, USA), Phosphate Buffered Saline (PBS), hypoxanthine, and xanthine oxidase from MilliporeSigma (Massachusetts, USA), KH2PO4, K2HPO4, Ethylenediaminetetraaceticacid (EDTA), ferric chloride,, Bovine Serum Albumin (BSA), glucose, fructose. and Sodium hydroxide (NaOH) from Thermo Fisher Scientific (Massachusetts, USA).

Cell Lines: Rat Cardiomyoblasts (H9c2) were obtained from ATCC (Virginia, USA). It is derived from embryonic BD1X rat heart tissue that exhibits many of the properties of skeletal muscle. Cells were maintained in Dulbecco's Modified Eagle's Medium (DMEM) from Thermofisher (MA, USA) supplemented with 10% Fetal Bovine Serum (FBS) from Thermofisher (MA, USA) and 1% Penicillin-Streptomycin (PS) from MilliporeSigma (MA, USA). Microglial Cells (IMG) were obtained from Kerafast (MA, USA). It is an immortalized microglial cell line isolated from the brains of adult mice. Cells were maintained in DMEM supplemented with 10% FBS and 1% PS.

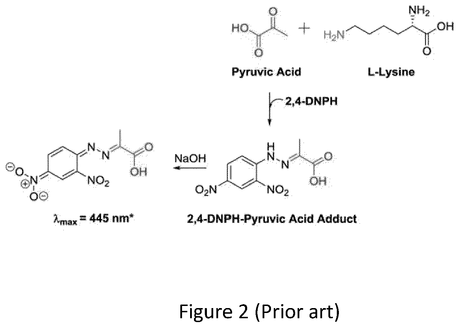

Glycation Reversal Assay: For CML cleavage reaction 90 µl of 100mM CML in PBS was incubated with 10µl ingredients diluted in PBS. Incubation was at 37°C and 300 rpm for 4 hours. After 4 hours, 50 µL of 2,4-DNPH solution (1 mm in 1 N HCl) was then added to the mixture, incubated at 37 °C for 10 min, with 350 µL of 0.6 N NaOH. Optical density was measured at 445 nm for a 150 µl volume. The readout of this CML -dissolution assay is based on the cleavage of CML which results in the formation of L-Lysine, hydrogen peroxide and pyruvic acid. Using the α-keto-acid derivatization assay described in, sodium pyruvate reacts with 2,4-DNPH to form an adduct that can be modified by a base and detected by measuring optical density. The chemical reaction shown by Kim et al is shown in Figure 2 taken from their publication.

Glycation Prevention Assay: Glycation prevention reaction Buffer contained following ingredients: 500 mL of phosphate buffer (KH2PO4 + K2HPO4, 0.1 M), EDTA (73mg), FeCl36H2O (33mg), Hypoxanthine(20.5mg). To Eppendorf microfuge tubes (1.5 mL) the following reaction mixtures were added: 94 µL BSA (16mg/ml in buffer) , 25 µL Glucose (1.67M in buffer), 10 µL Xanthine Oxidase (XO- 18 mU in buffer) , 10 µL Test ingredients (40mg/ml stock in DMSO), 882 µL buffer. Controls were set up lacking BSA, Glucose and Xanthine Oxidase (XO). Positive control lacked Test ingredient. Every reaction was set up in duplicate. Tubes were incubated in the dark at 37°C for 5 days with shaking at 450 rpm. After incubation, aliquots were transferred to black 96 well plates. Each reaction tube was aliquoted into 3 wells at 290 µL each to be analyzed in triplicate. Total fluorescence intensity was measured with excitation and maximum emission at 360 nm and 460 nm respectively.

Cell Protection from Oxidative Stress: IMG cells were grown to confluency and pretreated with test compounds for 24 hours at 37°C. Media was removed and cells were exposed to Hydrogen Peroxide for 1 hour at 37°C. After one hour, H₂O₂ was removed and cells were incubated in DMEM supplemented with 1% BSA for a further 24 hours at 37°C to allow them to stabilize. Subsequently, cell viability was assessed using Alamar Blue Cell Viability Reagent from Thermofisher (MA, USA). Alamar Blue Reagent is an oxidized form of redox indicator that is blue in color. When incubated with viable cells, the reagent changes color from blue to red and can be measured by absorbance at 570nm.

Mitobiogenesis: MitoBiogenesis^{™} In-Cell ELISA Kit (Colorimetric) was purchased from Abcam (Cambridge, UK). H9c2 Cells were grown to confluency in 96 well plates and treated with test ingredients for 24 hours at 37°C. Media was removed and cells were washed with Phosphate Buffered Saline provided in the kit. The cells were then fixed and processed as per the protocol provided with the kit. This assay was used to quantify two mitochondrial proteins: subunit I of Complex IV (COX-I) and the 70 kDa subunit of Complex II (SDH-A). No treatment control was used to show relative protein levels in each case.

Natural Compounds Promote Reversal of Glycation: To date there is no available therapy that can break down or repair fully developed AGEs. Our study focused on the basic biological process in protein glycation which involves Nε -(carboxymethyl)lysine (CML), which is lysine-derived AGEs. The presence of CML is linked to the progression of various diabetic complications and some neurodegenerative diseases. CML is one of the most abundant AGEs found in the renal compartment and is linked to the loss of kidney function in chronic kidney disease.

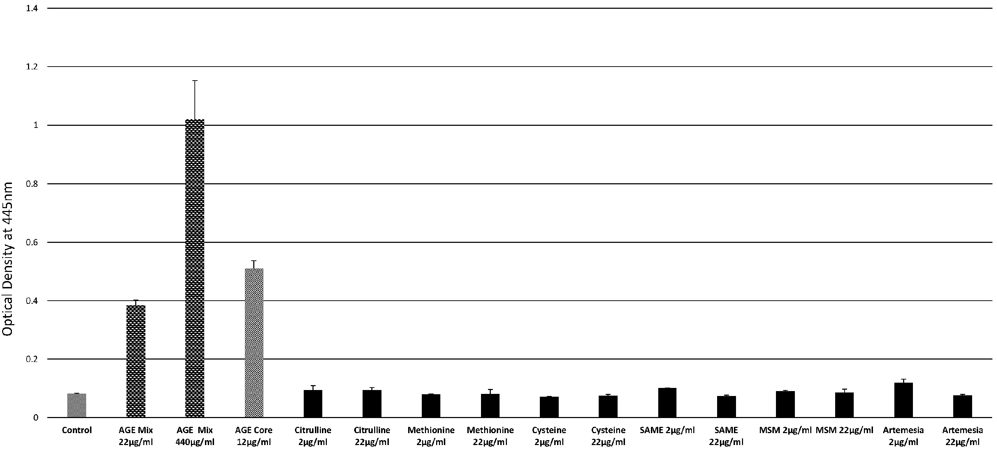

Earlier study indicated that MnmC, an enzyme involved in a bacterial tRNA-modification pathway, is capable of reversing carboxymethyl-lysine (CML) back to the native lysine structure. Here we applied various natural compounds and their mixes to investigate their efficacy in reversal of CML to native Lysine. Figure 3 shows reversal of CML to native lysine in the presence of individual compounds and their combinations (Mix and Core).

The results on Figure 3 show that select natural compounds tested individually at two different concentrations did not have significant effect on releasing free Lysine from CML. However, their combination in the Mix dramatically increased this process in the concentration dependent fashion. Application of Mix at the concentration of 22 mg/ml (containing each individual compounds at 2 mg/ml dose) resulted in about 4 times higher release of free lysine compared to control. In the presence of Mix at 440 mg/ml (containing each individual compounds at 40 mg/ml dose) this increase was even higher - about 10 times increase. A subset of compounds (Core) applied at 12 mg/ml (with each ingredient at 2 mg/ml dose) was also effective in increasing CME reversal process by about 500% compared to control.

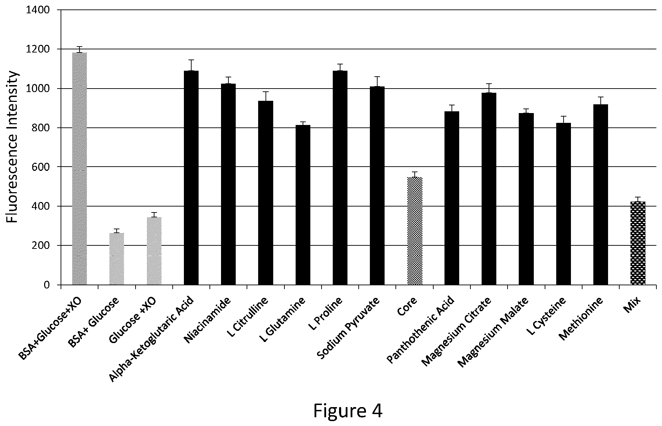

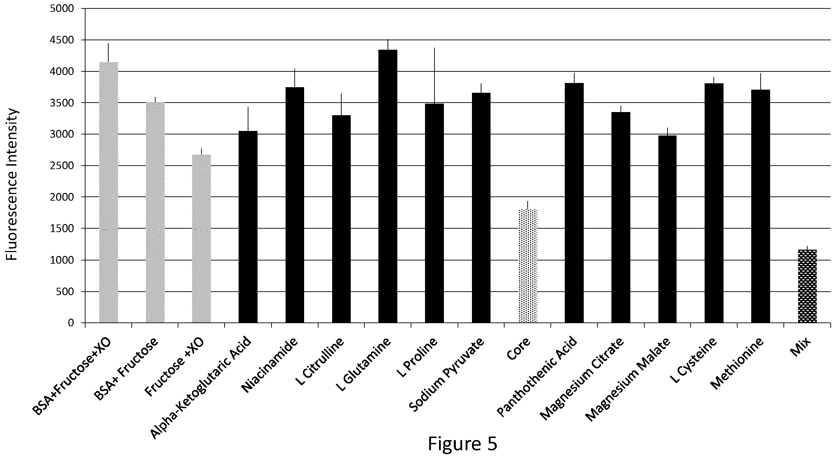

Natural Compounds Prevent Protein Glycation: The results on figure 4 and figure 5 show that incubation of BSA in the presence of Glucose and Fructose increases formation of AGE-BSA with Fructose (figure 5) causing more protein glycation than Glucose (Figure 4). Individual ingredients showed different efficacy in preventing the AGE-BSA formation. The highest efficacy in preventing glycation process was observed when these ingredients were applied in combinations either as the Mix and Core. As such, in glucose- driven glycation the Core combination was effective in preventing AGE-BSA by 54% and the Mix by 64% (Figure 4). Fructose promoted glycation of BSA was inhibited by the Core combination by 57% and by Mix by 73% (Figure 5).

Natural Compounds Promote Mitobiogenesis: Mitochondrial metabolism is a major source of bioenergy as most of cellular ATP is generated in the mitochondria. Cellular mitochondrial pool is inherited from our mothers and can increase only by growth and division of preexisting mitochondria. Both high mitochondrial activity and their number are essential in our body metabolism, in supporting cardiac and skeletal muscle functions, controlling diabetes, aging and many chronic diseases. Mitochondria are master regulators of insulin secretion, and their dysfunction has been implicated in the pathogenesis of insulin resistance, the hallmark of type 2 diabetes.

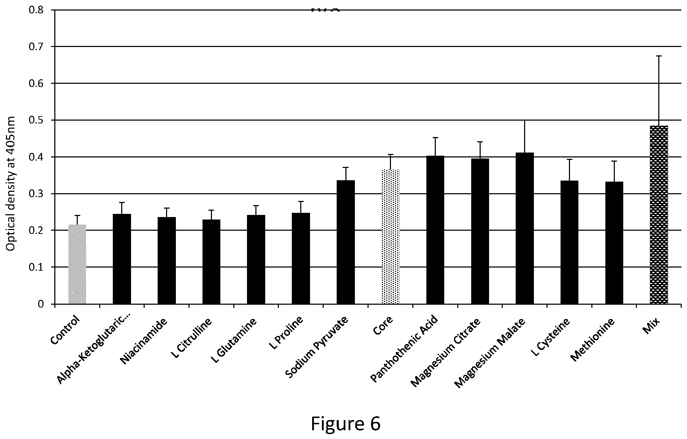

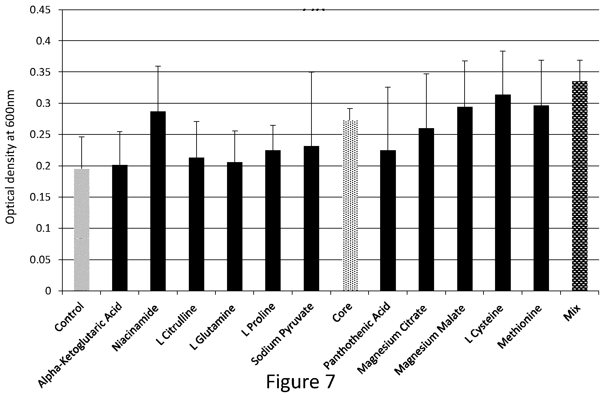

Mitochondrial biogenesis can be evaluated by changes in two mitochondrial enzymes: Succinate Dehydrogenase (SDH) which forms a part of mitochondrial Complex II A and Cyclooxygenase -1 (Cox-1) present in the mitochondrial Complex IV. We evaluated the effects of various natural compounds and their combinations as Mix and Core on the levels of SDH and COX-1 as a measure of mitobiogenesis in the heart muscle cells. The results are presented on figure 6 and figure 7.

The results on figure 6 shows that among individual compounds, sodium pyruvate, pantothenic acid, magnesium citrate and magnesium malate had the most pronounced stimulatory effects on SDH in the cardiac muscle cells by increasing its levels from 54% (pyruvate) to 86% (magnesium malate) compared to control. Highest stimulatory effect on SDH was observed when all test ingredients were combined as a Mix - 118% increase. Core ingredients stimulated SDH by 68% compared to control. Cox-1 levels in the heart muscle cells increased in the presence of niacinamide, magnesium malate, and methionine by 52-58% and cysteine by 41% compared to control (figure 7). The highest stimulatory effect on Cox1 levels was achieved in the presence of Mix (79%) with Core having more moderate effect of 42% increase.

Natural Compounds Protect Glial Cells from Oxidative Damage: Oxidative stress plays a pivotal role in the development of diabetes complications including diabetes-specific pathology in the peripheral nerves, retina, renal glomerulus, and accelerated atherosclerosis affecting arteries that supply blood to the heart, brain, and lower extremities. Oxidative stress is also implicated in development of cancer, neurodegenerative diseases (Alzheimer's and Parkinson") ALS, pulmonary diseases, and various allergies among other.

We tested protective effects of individual natural compounds and their combinations in microglial cells exposed to oxidative stress generated by hydrogen peroxide.

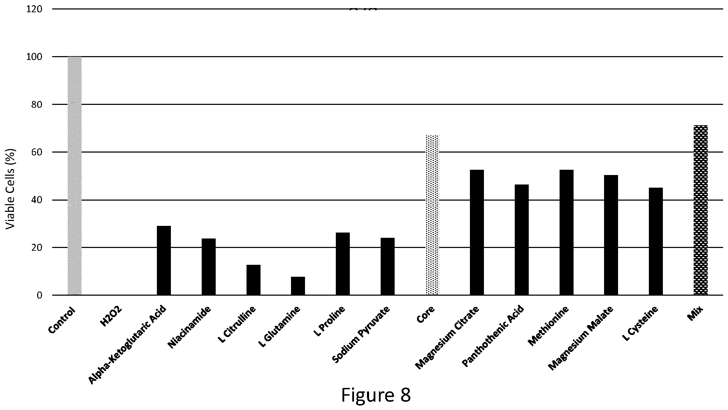

The results on figure 8 shows that in the presence of test individual ingredients the survival of cells exposed to hydrogen peroxide increased. Glial cells exposed to H₂O₂ in the presence of magnesium citrate, magnesium malate and methionine survived in about 50%. Other test compounds could protect cells in the range of 5% to 30%. The highest cell protection from oxidative stress (about 70% viable cells) was achieved when these nutrients were combined as Core and Mix. Control was cells untreated by any ingredient and not exposed to hydrogen peroxide, H₂O₂ indicates survival of cells exposed to hydrogen peroxide.

Drug formulations suitable for these administration routes can be produced by adding one or more pharmacologically acceptable carrier to the agent and then treating the micronutrient composition through a routine process known to those skilled in the art. The mode of administration includes, but is not limited to, non-invasive peroral, topical (for example, transdermal), enteral, transmucosal, targeted delivery, sustained-release delivery, delayed release, pulsed release and parenteral methods. Peroral administration may be administered both in liquid and dry state. In one embodiment, micronutrient composition would be more specifically Mix and Core. A pharmaceutical composition comprises of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg and is used as a nutritional supplement composition or as a pharmaceutical composition. There is a process for producing the micronutrient composition Mix and core comprising the steps of mixing the ingredients of the micronutrient composition and optionally formulating the micronutrient composition.

Formulations suitable for oral administration may be in the form of capsules, cachets, pills, tablets, lozenges (using flavored bases, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin or sucrose and acacia), each containing a predetermined amount of a subject composition as an active ingredient. Subject compositions may also be administered as a bolus, electuary or paste.

When an oral solid drug product is prepared, micronutrient composition is mixed with an excipient (and, if necessary, one or more additives such as a binder, a disintegrant, a lubricant, a coloring agent, a sweetening agent, and a flavoring agent), and the resultant mixture is processed through a routine method, to thereby produce an oral solid drug product such as tablets, coated tablets, granules, powder or capsules. Additives may be those generally employed in the art. Examples of excipients include lactate, sucrose, sodium chloride, glucose, starch, calcium carbonate, kaolin, microcrystalline cellulose and silicic acid. Binders include water, ethanol, propanol, simple syrup, glucose solution, starch solution, liquefied gelatin, carboxymethylcellulose, hydroxypropyl cellulose, hydroxypropyl starch, methyl cellulose, ethyl cellulose, shellac, calcium phosphate and polyvinyl pyrrolidone. Disintegrants include dried starch, sodium arginate, powdered agar, sodium hydroxy carbonate, calcium carbonate, sodium lauryl sulfate, monoglyceryl stearate and lactose. Lubricants include purified talc, stearic acid salts, borax and polyethylene glycol. Sweetening agents include sucrose, orange peel, citric acid and tartaric acid.

When a liquid drug product for oral administration is prepared, micronutrient composition is mixed with an additive such as a sweetening agent, a buffer, a stabilizer, or a flavoring agent, and the resultant mixture is processed through a routine method, to produce an orally administered liquid drug product such as an internal solution medicine, syrup or elixir. Examples of the sweetening agent include vanillin; examples of the buffer include sodium citrate; and examples of the stabilizer include tragacanth, acacia, and gelatin.

For the purposes of transdermal (e.g., topical) administration, dilute sterile, aqueous or partially aqueous solutions (usually in about 0.1% to 5% concentration), otherwise similar to the above parenteral solutions, may be prepared with micronutrient composition.

Formulations containing micronutrient composition for rectal or vaginal administration may be presented as a suppository, which may be prepared by mixing a subject composition with one or more suitable non-irritating carriers, comprising, for example, cocoa butter, polyethylene glycol, a suppository wax or a salicylate, which is solid at room temperature, but liquid at body temperature and, therefore, will melt in the appropriate body cavity and release the encapsulated compound(s) and composition(s). Formulations that are suitable for vaginal administration also include pessaries, tampons, creams, gels, pastes, foams or spray formulations containing such carriers as are known in the art to be appropriate.

A targeted-release portion for capsules containing micronutrient composition can be added to the extended-release system by means of either applying an immediate-release layer on top of the extended release core; using coating or compression processes, or in a multiple-unit system such as a capsule containing extended- and immediate- release beads.

When used with respect to a micronutrient composition, the term "sustained release" is art recognized. For example, a therapeutic composition that releases a substance over time may exhibit sustained-release characteristics, in contrast to a bolus type administration in which the entire amount of the substance is made biologically available at one time. In particular embodiments, upon contact with body fluids, including blood, spinal fluid, mucus secretions, lymph or the like, one or more of the pharmaceutically acceptable excipients may undergo gradual or delayed degradation (e.g., through hydrolysis), with concomitant release of any material incorporated therein, e.g., a therapeutic and/or biologically active salt and/or composition, for a sustained or extended period (as compared with the release from a bolus). This release may result in prolonged delivery of therapeutically effective amounts of any of the therapeutic agents disclosed herein.

Current efforts in the area of drug delivery include the development of targeted delivery, in which the drug is only active in the target area of the body (for example, mucous membranes such as in the nasal cavity), and sustained-release formulations, in which the micronutrient composition is released over a period of time in a controlled manner from a formulation. Types of sustained release formulations include liposomes, drug-loaded biodegradable microspheres and micronutrient composition polymer conjugates.

Delayed-release dosage formulations are created by coating a solid dosage form with a film of a polymer, which is insoluble in the acid environment of the stomach, but soluble in the neutral environment of the small intestine. The delayed-release dosage units can be prepared, for example, by coating a micronutrient composition with a selected coating material. The micronutrient composition may be a tablet for incorporation into a capsule, a tablet for use as an inner core in a "coated core" dosage form, or a plurality of drug-containing beads, particles or granules, for incorporation into either a tablet or a capsule. Preferred coating materials include bioerodible, gradually hydrolysable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract, or slowly erode as the dosage form passes through the gastrointestinal tract, while enzymatically degradable polymers are degraded by bacterial enzymes present in the lower gastrointestinal tract, particularly in the colon. Alternatively, a delayed-release tablet may be formulated by dispersing a drug within a matrix of a suitable material such as a hydrophilic polymer or a fatty compound. Suitable hydrophilic polymers include, but are not limited to, polymers or copolymers of cellulose, cellulose ester, acrylic acid, methacrylic acid, methyl acrylate, ethyl acrylate and vinyl or enzymatically degradable polymers or copolymers as described above. These hydrophilic polymers are particularly useful for providing a delayed-release matrix. Fatty compounds for use as a matrix material include, but are not limited to, waxes (e.g., carnauba wax) and glycerol tristearate. Once the active ingredient is mixed with the matrix material, the mixture can be compressed into tablets.

A pulsed-release dosage is one that mimics a multiple dosing profile without repeated dosing, and typically allows at least a twofold reduction in dosing frequency as compared with the drug presented as a conventional dosage form (e.g., as a solution or prompt drug-releasing, conventional solid dosage form). A pulsed-release profile is characterized by a time period of no release (lag time) or reduced release, followed by rapid drug release. These can be formulated for critically ill patients using the instant micronutrient composition.

The phrases "parenteral administration" and "administered parenterally" as used herein refer to modes of administration other than enteral and topical, such as injections, and include without limitation intravenous, intramuscular, intrapleural, intravascular, intrapericardial, intraarterial, intrathecal, intracapsular, intraorbital, intracardiac, intradermal, intraperitoneal, transtracheal, subcutaneous, subcuticular, intra-articular, subcapsular, subarachnoid, intraspinal and intrastemal injection and infusion.

Certain micronutrient composition disclosed herein, suitable for parenteral administration, comprise one or more subject compositions in combination with one or more pharmaceutically acceptable sterile, isotonic, aqueous, or non-aqueous solutions, dispersions, suspensions or emulsions, or sterile powders, which may be reconstituted into sterile injectable solutions or dispersions just prior to use, and which may contain antioxidants, buffers, bacteriostats, solutes that render the formulation isotonic within the blood of the intended recipient, or suspending or thickening agents.

When an injection product is prepared, micronutrient composition is mixed with an additive such as a pH regulator, a buffer, a stabilizer, an isotonicity agent or a local anesthetic, and the resultant mixture is processed through a routine method, to thereby produce an injection for subcutaneous injection, intramuscular injection, or intravenous injection. Examples of the pH regulator or buffer include sodium citrate, sodium acetate and sodium phosphate; examples of the stabilizer include sodium pyrosulfite, EDTA, thioglycolic acid, and thiolactic acid; examples of the local anesthetic include procaine hydrochloride and lidocaine hydrochloride; and examples of the isotonicity agent include sodium chloride and glucose.

Adjuvants are used to enhance the immune response. Various types of adjuvants are available. Haptens and Freund's adjuvant may also be used to produce water-in-oil emulsions of immunogens.

The phrase "pharmaceutically acceptable" is art recognized. In certain embodiments, the term includes compositions, polymers and other materials and/or dosage forms that are within the scope of sound medical judgment, suitable for use in contact with the tissues of mammals, both human beings and animals, without excessive toxicity, irritation, allergic response or other problem or complication, commensurate with a reasonable benefit-risk ratio.

The phrase "pharmaceutically acceptable carrier" is art recognized, and includes, for example, pharmaceutically acceptable materials, compositions or vehicles, such as a liquid or solid filler, diluent, solvent or encapsulating material involved in carrying or transporting any subject composition from one organ or portion of the body, to another organ or portion of the body. Each carrier must be "acceptable" in the sense of being compatible with the other ingredients of a subject composition, and not injurious to the patient. In certain embodiments, a pharmaceutically acceptable carrier is non-pyrogenic. Some examples of materials that may serve as pharmaceutically acceptable carriers include: (1) sugars, such as lactose, glucose and sucrose; (2) starches, such as corn starch and potato starch; (3) cellulose and its derivatives, such as sodium carboxymethyl cellulose, ethyl cellulose and cellulose acetate; (4) powdered tragacanth; (5) malt; (6) gelatin; (7) talc; (8) cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

In certain embodiments, the micronutrient compositions described herein are formulated in a manner such that said compositions will be delivered to a mammal in a therapeutically effective amount, as part of a prophylactic, preventive or therapeutic treatment to overcome the infection caused by corona viruses (irrespective of the type).

In certain embodiments, the dosage of the micronutrient compositions, which may be referred to as therapeutic composition provided herein, may be determined by reference to the plasma concentrations of the therapeutic composition or other encapsulated materials. For example, the blood samples may be tested for their immune response to their corresponding viral load or lack thereof.

The therapeutic micronutrient composition provided by this application may be administered to a subject in need of treatment by a variety of conventional routes of administration, including orally, topically, parenterally, e.g., intravenously, subcutaneously or intramedullary. Further, the therapeutic compositions may be administered intranasally, as a rectal suppository, or using a "flash" formulation, i.e., allowing the medication to dissolve in the mouth without the need to use water. Furthermore, the compositions may be administered to a subject in need of treatment by controlled-release dosage forms, site-specific drug delivery, transdermal drug delivery, patch-mediated drug delivery (active/passive), by stereotactic injection, or in nanoparticles.

Expressed in terms of concentration, an active ingredient can be present in the therapeutic compositions of the present invention for localized use via the cutis, intranasally, pharyngolaryngeally, bronchially, intravaginally, rectally or ocularly.

For use as aerosols, the active ingredients can be packaged in a pressurized aerosol container together with a gaseous or liquefied propellant, for example dichlorodifluoromethane, carbon dioxide, nitrogen, propane and the like, with the usual adjuvants such as cosolvents and wetting agents, as may be necessary or desirable. The most common routes of administration also include the preferred transmucosal (nasal, buccal/sublingual, vaginal, ocular and rectal) and inhalation routes.

In addition, in certain embodiments, the subject micronutrient composition of the present application may be lyophilized or subjected to another appropriate drying technique such as spray drying. The subject compositions may be administered once, or may be divided into a number of smaller doses to be administered at varying intervals of time, depending in part on the release rate of the compositions and the desired dosage.

Formulations useful in the methods provided herein include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of a subject micronutrient composition that may be combined with a carrier material to produce a single dose may vary depending upon the subject being treated and the particular mode of administration. Physiological dose levels for mammalian consumption were calculated based on various factors which include type of administration, species dependency and mode of action, such as transdermal vs oral. The range disclosed includes those factors along with scientific calculations. The range may differ within the range as well depending on formulations and species. Drug formulations suitable for these administration routes can be produced by adding one or more pharmacologically acceptable carrier to the agent and then treating the micronutrient composition through a routine process known to those skilled in the art. The mode of administration includes, but is not limited to, non-invasive peroral, topical (for example, transdermal), enteral, transmucosal, targeted delivery, sustained-release delivery, delayed release, pulsed release and parenteral methods. Peroral administration may be administered both in liquid and dry state.

The therapeutically acceptable amount described herein may be administered in inhalant or aerosol formulations. The inhalant or aerosol formulations may comprise one or more agents, such as adjuvants, diagnostic agents, imaging agents, or therapeutic agents useful in inhalation therapy. The final aerosol formulation may, for example, contain 0.005-90% w/w, for instance 0.005-50%, 0.005-5% w/w, or 0.01-1.0% w/w, of medicament relative to the total weight of the formulation.

Examples of suitable aqueous and non-aqueous carriers that may be employed in the micronutrient composition include water, ethanol, polyols (such as glycerol, propylene glycol, polyethylene glycol and the like), and suitable mixtures thereof, vegetable oils such as olive oil, and injectable organic esters such as ethyl oleate. Proper fluidity may be maintained, for example by the use of coating materials such as lecithin, by the maintenance of the required particle size in the case of dispersions, and by the use of surfactants.

### EMBODIMENTS

The following are preferred embodiments of the invention. The respective features can be freely combined with each other and with the claims without departing from the present invention.
1. A micronutrient composition, comprising of:
   micronutrient composition comprises of alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, Methionine, S- adenosyl-methionine (SAMe), Methylsulfonylmethane (MSM), and Wormwood powder or extract.and optionally adding one or combination of pharmaceutically acceptable carriers or excipient or liquefied propellant or buffer or pH regulator or stabilizer or coating or flavoring agent optionally formulated as a tablet, coated tablet, capsule, pill, intranasal, lozenges, emulsion, pastilles, suppository, paste or injectable solution.
2. The micronutrient composition of embodiment 1, further consisting of the alpha-Ketoglutaric Acid 10 mg-30000 mg, Niacinamide or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder or extract 1 mg-10000 mg.
3. The micronutrient composition of embodiment 1, further consisting of the alpha-Ketoglutaric Acid, Niacinamide or Niacin, L-Proline, L-Glutamine, L-Citrulline, Sodium Pyruvate, Pantothenic Acid, L-Cysteine or N-acetyl Cysteine, Magnesium Malate, Magnesium Citrate, and Methionine.
4. The micronutrient composition of embodiment 3, further consisting of the alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg.
5. The micronutrient composition of embodiment 4, wherein the micronutrient composition is formulated for treating a human to reverse an antioxidant damage in a specific disease.
6. The micronutrient mixture of embodiment 5, wherein the specific disease is diabetes.
7. The micronutrient composition of embodiment 4, wherein the micronutrient composition is formulated for improving, protecting and mitigating from a specific disease that is initiated by an antioxidant cellular reaction.
8. The micronutrient composition of embodiment 7, wherein the specific disease is a diabetes, cardiovascular disease, Alzheimer's and inflammation which accelerates aging of tissues.
9. A method of treating an antioxidant prone disease, comprising;
   administering to a human in need thereof a micronutrient composition, wherein the micronutrient composition comprises of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg and optionally one or a combination of pharmaceutically acceptable carriers or excipient or liquefied propellant, buffer, pH regulator, stabilizer, coating or flavoring agent, wherein the micronutrient composition is formulated as a tablet, coated tablet, capsule, pill, intranasal, lozenges, emulsion, pastilles, suppository, paste or injectable solution.
10. The method of embodiment 9, further comprising;
   decreasing formation of an advanced glycation end product.
11. The method of embodiment 9, further comprising;
   Reversing formation of an advanced glycation end product, wherein the advanced glycation end product is caused by excess glucose in the human.
12. The method of embodiment 9, further comprising;
   promoting mitobiogenesis in the muscle cells and protecting microglial cells from death caused by oxidative stress.
13. The method of embodiment 9, further comprising;
   lowering a level protein glycation in human.
14. A method of using a micronutrient composition, comprising;
   administering to a human in need thereof the micronutrient composition, wherein the micronutrient compound is a mix of alpha-Ketoglutaric Acid 10 mg-30000 mg, Niacinamide or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine or N-acetyl Cysteine 10 mg-20000 mg, Magnesium Malate 10 mg-20000 mg, Magnesium Citrate 10 mg-30000 mg, and Methionine 10 mg-20000 mg, wherein the micronutrient composition is formulated as a tablet, coated tablet, capsule, pill, intranasal, lozenges, emulsion, pastilles, suppository, paste or injectable solution and optionally adding one or a combination of pharmaceutically acceptable carriers or excipient or liquefied propellant, buffer, pH regulator, stabilizer, coating or flavoring agent; and
   treating a human suffering from an oxidative stress induced disease by reducing a glycation optionally protein and glucose.

## Claims

1. A micronutrient composition, comprising or consisting of at least 6 ingredients, for example alpha-Ketoglutaric Acid, Niacinamide and/or Niacin, L-Proline, L-Glutamine, L-Citrulline and Sodium Pyruvate, preferably at least 11 ingredients, for example alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate, Magnesium Citrate and Methionine, more preferably all of the ingredients which are selected from the group consisting of:
alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate and/or Magnesium Citrate, Methionine and/or S- Adenosyl-Methionine (SAMe), Methylsulfonylmethane (MSM), and Wormwood powder and/or extract,
the micronutrient composition optionally additionally containing one or a combination of pharmaceutically acceptable carrier or excipient or liquefied propellant or buffer or pH regulator or stabilizer or coating or flavoring agent or formulation adjuvant,
the micronutrient composition optionally being formulated as a tablet, coated tablet, capsule, pill, intranasal, lozenge, emulsion, pastille, suppository, paste or injectable solution.

2. The micronutrient composition of claim 1, in which the ingredients, if present, are contained in the following amounts: alpha-Ketoglutaric Acid 10 mg-30000 mg, Niacinamide and/or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder and/or extract 1 mg-10000 mg.

3. The micronutrient composition of claim 1 or 2, consisting of alpha-Ketoglutaric Acid, Niacinamide and/or Niacin, L-Proline, L-Glutamine, L-Citrulline, Sodium Pyruvate, Pantothenic Acid, L-Cysteine and/or N-acetyl Cysteine, Magnesium Malate and/or Magnesium Citrate, and Methionine, and optionally additionally one or a combination of pharmaceutically acceptable carrier or excipient or liquefied propellant or buffer or pH regulator or stabilizer or coating or flavoring agent.

4. The micronutrient composition of one of claims 1 to 3, in which the ingredients, if present, are contained in the following amounts: alpha-Ketoglutaric Acid 10 mg-30000 mg, Niacinamide and/or Niacin 1mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, and Methionine 10 mg-20000 mg.

5. The micronutrient composition of one of claims 1 to 4, wherein the dosage form is one oral capsule once a day, twice a day or three times a day.

6. The micronutrient composition of one of claims 1 to 5, wherein the formulation adjuvant is selected from the group consisting of a binder, a disintegrant, a lubricant, a coloring agent, a sweetening agent, a flavoring agent, and mixtures thereof.

7. The use of the micronutrient composition of one of claims 1 to 6 in a pharmaceutical composition.

8. A pharmaceutical composition, containing the micronutrient composition of one of claims 1 to 6 and optionally a pharmaceutically acceptable carrier.

9. The pharmaceutical composition of claim 8 for use in the prevention or treatment of diabetes, cardiovascular disease, Alzheimer's disease or inflammation.

10. A process for preparing the micronutrient composition of one of claims 1 to 6 or the pharmaceutical composition of one of claims 8 or 9 by mixing the ingredients of the micronutrient composition of one of claims 1 to 6 or the pharmaceutical composition of one of claims 8 or 9 and optionally bringing the mixture into the desired dosage form.

11. The process of claim 10, comprising mixing the ingredients, if present, in the following amounts: alpha-Ketoglutaric Acid 10 mg-30000 mg, Niacinamide and/or Niacin 1 mg-10000 mg, L-Proline 10 mg-30000 mg, L-Glutamine 10 mg-50000 mg, L-Citrulline 10 mg-20000 mg, Sodium Pyruvate 10 mg-50000 mg, Pantothenic Acid 0.1 mg-200 mg, L-Cysteine and/or N-acetyl Cysteine 10 mg-20000 mg, Magnesium Malate 10 mg-20000 mg, Magnesium Citrate 10 mg-30000 mg, Methionine 10 mg-20000 mg, S- adenosyl-methionine (SAMe) 1 mg-20000 mg, Methylsulfonylmethane (MSM) 1 mg-20000 mg, and Wormwood powder and/or extract 1 mg-10000 mg.

12. The process of claim 10 or 11, comprising formulating the micronutrient composition as a tablet, coated tablet, capsule, pill, intranasal, lozenge, emulsion, pastille, suppository, paste or injectable solution.
